(19) Europäisches Patentamt
European Patent Office
Office européen des brevets

(11) **EP 4 437 964 A1**

(12) **EUROPEAN PATENT APPLICATION**
published in accordance with Art. 153(4) EPC

(43) Date of publication:
**02.10.2024 Bulletin 2024/40**

(21) Application number: **22898231.0**

(22) Date of filing: **30.09.2022**

(51) International Patent Classification (IPC):
***A61B 5/372*** (2021.01)

(52) Cooperative Patent Classification (CPC):
**A61B 5/372**

(86) International application number:
**PCT/JP2022/036874**

(87) International publication number:
**WO 2023/095446 (01.06.2023 Gazette 2023/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.11.2021 JP 2021191603**

(71) Applicant: **CYBERDYNE INC.**
**Tsukuba-shi, Ibaraki 305-0818 (JP)**

(72) Inventors:
• **SANKAI, Yoshiyuki**
**Tsukuba-shi, Ibaraki 305-0818 (JP)**
• **YOSHIBA, Takumu**
**Tsukuba-shi, Ibaraki 305-8577 (JP)**

(74) Representative: **Mewburn Ellis LLP**
**Aurora Building**
**Counterslip**
**Bristol BS1 6BX (GB)**

(54) **BRAIN ACTIVITY DETECTION DEVICE AND BRAIN ACTIVITY DETECTION METHOD**

(57)    An EEG signal of a single channel indicating frontal lobe EEGs is detected via a pair of electrodes placed at a test subject's forehead; data is converted to segmented images expressed with a two-dimensional coordinate system whose coordinate axes are time and amplitude; and then the relevant seizure symptoms are estimated by referring to a seizure symptom estimation model constructed by deep learning of EEG characteristic data in the respective segmented images.

FIG. 1

1 Brain Activity Detection System

EP 4 437 964 A1

**Description**

TECHNICAL FIELD

The present invention relates to a brain activity detection device and a brain activity detection method and aims at proposing, particularly, a brain activity detection device and brain activity detection method for detecting an epileptic seizure(s) in everyday life.

BACKGROUND ART

Epilepsy is a neurological disorder that suddenly causes a seizure due to abnormal excitement of neurons in the brain. According to the World Health Organization (WHO), it is estimated that there are approximately 50 million epilepsy patients all over the world. There are a wide variety of seizure symptoms such as tonic-clonic seizures, absence seizures, myoclonic seizures, and atonic seizures.

Many patients can live their lives without seizures if they receive appropriate diagnosis and medical treatment; however, the current situation is that approximately 30% of the patients cannot control the seizures with medicines, which hinders their social lives. Also, when a seizure occurs, there is a risk that a patient may hit something or drown in a bath and, therefore, a caregiver needs to ensure the patient's safety.

However, the seizure can happen anytime and anywhere, so that the caregiver has to always accompany the patient and there is fear that their everyday lives may be limited. Therefore, a seizure detection system is required in order to enhance the quality of lives of the patient(s) and the caregiver(s).

A seizure detection method using multi-channel EEG (electroencephalograms) (see NPL 1) has been conventionally proposed as the above-described seizure detection system. Moreover, there are also proposed an EEG recording analysis method on the premise of multi-channels (see PTL 1) and a method for identifying a pathological brain activity pattern of a target by using a nonlinear classification method (see PTL 2).

CITATION LIST

PATENT LITERATURE

PTL 1: Japanese Patent Application Laid-Open (Kokai) Publication No. 2017-80483
PTL 2: Japanese Unexamined Patent Application Publication (Translation of PCT Application) No. 2020-512860

NON-PATENT LITERATURE

NPL 1: Zabihi M, Kiranyaz S, Ince T, Gabbouj M (2013) Patient-specific epileptic seizure detection in long-term eeg recording in paediatric patients with intractable seizures

SUMMARY OF THE INVENTION

PROBLEMS TO BE SOLVED BY THE INVENTION

The seizure detection method using the multi-channel EEG as indicated in NPL 1 can detect seizures with higher accuracy than single channel EEG by using a method for extracting statistical characteristics and frequency domain characteristics.

However, an EEG measuring apparatus itself is of a relatively large size and weighs relatively heavy and electrodes need to be set all over the head, so the EEGs are normally measured inside a hospital and the problem is that a measurement location is limited.

Similarly, the EEG measuring methods using the multi-channel EEG according to PTL 2 and PTL 3 also have a problem that it is very difficult to always measure an EEG signal of a test subject in everyday life.

The present invention was devised in consideration of the above-described circumstance and aims at proposing a brain activity detection device and the brain activity detection method capable of effectively detecting a seizure(s) caused by brain activities of a test subject in everyday life.

MEANS TO SOLVE THE PROBLEMS

In order to solve the above-described problems, a brain activity detection device according to the present invention includes: a signal detection unit that detects an EEG signal of a single channel indicating frontal lobe EEGs via a pair of electrodes placed at a test subject's forehead; an image conversion unit that extracts partial time serieses from the EEG signal detected by the signal detection unit while sequentially dividing the EEG signal by a specified time width to make the partial time serieses partially overlap each other in a time direction, and performs data conversion of the respective partial time serieses to segmented images expressed with a two-dimensional coordinate system whose coordinate axes are time and amplitude; an EEG characteristics extraction unit that sequentially analyzes the segmented images converted by the image conversion unit and extracts each piece of EEG characteristic data in each of the segmented images; and a seizure symptom estimation unit that, while referring to a seizure symptom estimation model constructed by deep learning by using, as teacher data, EEG characteristic patterns classified according to respective seizure symptoms caused by human anomaly EEGs, estimates a relevant seizure symptom from the EEG characteristic data sequentially extracted by the EEG characteristics extraction unit.

As a result, the brain activity detection device can effectively detect a seizure(s) caused by the brain activities by placing the electrodes only at the test subject's forehead so as not to hinder everyday life, and using frontal lobe EEGs of one channel.

Moreover, according to the present invention, the image conversion unit extracts the partial time serieses from the EEG signal detected by the signal detection unit by using a sliding window method, while sequentially dividing the EEG signal by a sliding window at specified time intervals with overlaps at a specified ratio, and sets the respective partial time serieses in the two-dimensional coordinate system, which is enlarged within a range of a specified multiple of the extracted partial time serieses in an amplitude direction, and converts the partial time serieses to the segmented images.

As a result, when converting the EEG signal to the segmented images according to the partial time serieses, the brain activity detection device clarifies distinction between normal EEGs and anomaly EEGs and performs data processing to make it easier to identify EEG patterns from the images, so that it becomes possible to significantly enhance the accuracy for the EEG characteristics extraction unit to extract each piece of the EEG characteristic data in each analyzed segmented image. Particularly, noise caused by head movements, facial muscle tension, blinks, and so on is superimposed on the EEGs and the evaluation in one sliding window would not be sufficient to classify normal EEGs and anomaly EEGs, so that the brain activity detection device can solve such insufficiency.

Furthermore, according to the present invention, the EEG characteristics extraction unit sequentially judges a timing to change between an ictal period indicating a time period during a seizure and including a specified amount of time before and after the seizure and an interictal period indicating a time period after the ictal period until a next ictal period by using a specified seizure detection algorithm when extracting each piece of the EEG characteristic data in each of the segmented images.

As a result, the brain activity detection device can distinguish between the interictal period(s) which occupies most of the test subject's everyday life, and the ictal period(s) which is the time period including the specified amount of time during an actual seizure and before and after the seizure.

Furthermore, according to the present invention, the seizure symptom estimation unit undersamples the EEG characteristic data corresponding to the interictal period in accordance with a quantity of the EEG characteristic data corresponding to the ictal period with respect to the EEG characteristic data in the respective segmented images extracted by the EEG characteristics extraction unit, then creates n pieces of subdatasets, and estimates the relevant seizure symptom, by using each of classifiers according to each piece of the subdatasets, based on final output decided by taking a majority vote of outputs from all the classifiers.

As a result, since the ictal period is very short as compared to the interictal period(s) which occupies most of the test subject's everyday life, the brain activity detection device can balance ratios of data obtained during the interictal period(s) and the ictal period and can prevent overfitting (overlearning) in learning by using the classifiers and also prevent degradation of classification accuracy which is the learning result.

Furthermore, according to the present invention, the seizure symptom estimation unit applies a residual network as the classifier and applies backpropagation as a method for updating the teacher data for the seizure symptom estimation model.

As a result, by using the residual network to separate an input feature value from a residual feature value in advance, the brain activity detection device can transfer the feature value, which has been supplied from an upstream layer, directly without any change to a downstream layer even if the residual feature value cannot be calculated correctly; and it becomes possible to avoid a phenomenon that would hinder the progress of learning such as vanishing gradient which may occur at each layer existing on the upstream side of an intermediate layer. As a result, it is possible to suppress degradation of the feature values due to repeated operations associated with deepening of the network.

Furthermore, according to the present invention, a brain activity detection method includes: a first step of detecting an EEG signal of a single channel indicating frontal lobe EEGs via a pair of electrodes placed at a test subject's forehead; a second step of extracting partial time serieses from the EEG signal detected by the first step while sequentially dividing the EEG signal by a specified time width to make the partial time serieses partially overlap each other in a time direction, and performing data conversion of the respective partial time serieses to segmented images expressed with a two-dimensional coordinate system whose coordinate axes are time and amplitude; a third step of sequentially analyzing the segmented images converted by the second step and extracting each piece of EEG characteristic data in each of the segmented images; and a fourth step of, while referring to a seizure symptom estimation model constructed by deep learning by using, as teacher data, EEG characteristic patterns classified according to respective seizure symptoms caused by human anomaly EEGs, estimating a relevant seizure symptom from the EEG characteristic data sequentially extracted by the third step.

As a result, the brain activity detection method can effectively detect a seizure(s) caused by the brain activities by placing the electrodes only at the test subject's forehead so as not to hinder everyday life, and using frontal lobe EEGs of one channel.

ADVANTAGEOUS EFFECTS OF THE INVENTION

The brain activity detection device and brain activity detection method capable of detecting a seizure(s) caused by brain activities even in everyday life can be implemented according to the present invention.

BRIEF DESCRIPTION OF DRAWINGS

Fig. 1 is a conceptual diagram for explaining a brain activity detection system according to this embodiment;
Fig. 2 is a block diagram illustrating an internal structure of the brain activity detection device according to this embodiment;
Fig. 3 is a schematic diagram illustrating an arrangement status of electrode positions (Fp1-F7, Fp2-F8) of a signal channel;
Fig. 4 is a schematic diagram for explaining an ictal period and an interictal period(s);
Fig. 5 is a chart indicating the types of abnormal EEGs which appear during an epileptic seizure;
Fig. 6 is a schematic diagram for explaining an image conversion method using a sliding window method;
Fig. 7 is a conceptual diagram for explaining a seizure detection algorithm;
Fig. 8 is a conceptual diagram for explaining an architecture of a seizure symptom estimation model;
Fig. 9 is a chart indicating information of epilepsy patients who are test subjects;
Fig. 10 is a chart indicating EEG characteristic data corresponding to an ictal period of ID number chb01;
Fig. 11 is a chart indicating EEG characteristic data corresponding to an ictal period of ID number chb05;
Fig. 12 is a chart indicating experiment results of using the Fp1-F7 channel (Fig. 3);
Fig. 13 is a chart indicating experiment results of using the Fp2-F8 channel (Fig. 3);
Fig. 14 is a chart indicating results of selecting optimum channels in terms of sensitivity and specificity; and
Fig. 15 is a chart indicating radiographic image interpretation result examples of EEG characteristic data of patients.

DESCRIPTION OF EMBODIMENTS

One embodiment of the present invention will be described below in detail with reference to the drawings.

(1) Brain Activity Detection System According to This Embodiment

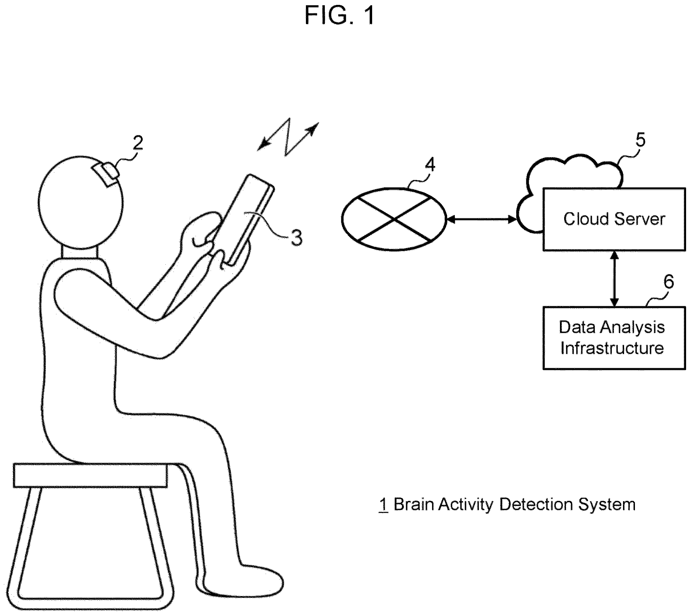

Fig. 1 shows the configuration of a brain activity detection system 1 according to the present invention. The brain activity detection system 1 has a brain activity detection device 2 for detecting frontal lobe EEGs via a pair of electrodes 2A placed on the forehead of a test subject, a terminal device 3 for receiving estimated data of seizure symptoms obtained as a detection result from the brain activity detection device 2, and an external cloud server 5 to which the estimated data is transmitted from the terminal device 3 via a network 4.

The brain activity detection device 2 is attached to a test subject's forehead via the electrodes 2A, the estimated data of seizure symptoms detected by the brain activity detection device 2 are received by the terminal device 3, and the terminal device 3 integrally manages the estimated data.

The terminal device 3 is composed of a wireless information terminal such as a smartphone and is designed to enable wireless connection with the brain activity detection device 2 via wireless communication such as a wireless LAN and to be connectable to the external cloud server 5 via the network 4.

After user authentication of the test subject and device authentication of the corresponding brain activity detection device 2 are performed on the cloud server 5, the estimated data of seizure symptoms obtained from the test subject(s) are accumulated in a database (which is not illustrated in the drawing). Then, a data analysis infrastructure 6 connected to the cloud server 5 analyzes the estimated data, which have been accumulated regarding each test subject and read from the database by a specified analysis method, and then manages the analysis results.

In fact, the data analysis infrastructure 6 performs statistical processing and big data analysis on the estimated data obtained from the test subject to perform inference and learning for QOL improvement (especially improvement of brain activities), and then stores and manages it in the database on the cloud server 5.

In this way, with the brain activity detection system 1, the test subject can use the terminal device 3 to receive, as feedback, analysis information of the estimated data of seizure symptoms of the test subject themselves from the cloud server 5 as necessary.

(2) Brain Activity Detection Device According to This Embodiment

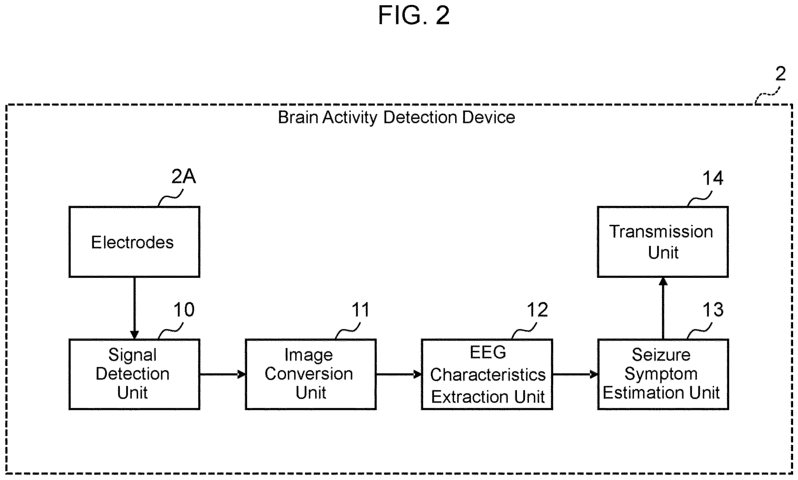

Fig. 2 illustrates the configuration of the brain activity detection device 2 according to the present invention. The brain activity detection device 2 is configured from a pair of electrodes 2A placed on the forehead of a test subject, a signal detection unit 10, an image conversion unit 11, an EEG characteristics extraction unit 12, a seizure symptom estimation unit 13, and a transmission unit 14 for transmitting the estimated data of seizure symptoms via wireless communication to the terminal device 3 (Fig. 1).

The signal detection unit 10 detects a single-channel EEG signal indicating the frontal lobe EEGs via the pair of electrodes 2A placed on the forehead of the test subject.

In the present invention, attention is focused on the frontal lobe EEGs of the test subject. The frontal association area is an area of the brain that receives information from other association areas and makes action plans based on the input. Also, it is interconnected by fibers with the motor area, the nucleus basalis, and the cerebral limbic system and receives information about movements, memories, arousals, regulations of autonomic nerves, etc.

Therefore, when convulsive symptoms such as unconscious twitching or loss of consciousness occur, it is assumed that abnormal neuronal excitations propagate to the frontal association area. Moreover, since the frontal association area is interconnected with other areas of the brain, when a local seizure occurs in another area, it is assumed that abnormal neuronal excitations may sometimes propagate to the frontal association area.

In addition, the skin of the test subject's forehead is exposed, making it easier to place the electrodes than at other measurement points. From these points of view, the frontal lobe EEGs of the test subject are considered to be the most suitable for detecting epileptic seizures in everyday life.

In this embodiment, the CHB-MIT Scalp EEG database was used as an EEG database included in the signal detection unit 10. In this EEG database, EEG data of pediatric patients with refractory seizures were measured by using the 10-20 (ten-twenty) method (a method of placing electrodes on the head during EEG measurements) recommended by the International Federation of Societies for Electroencephalography and Clinical Neurophysiology.

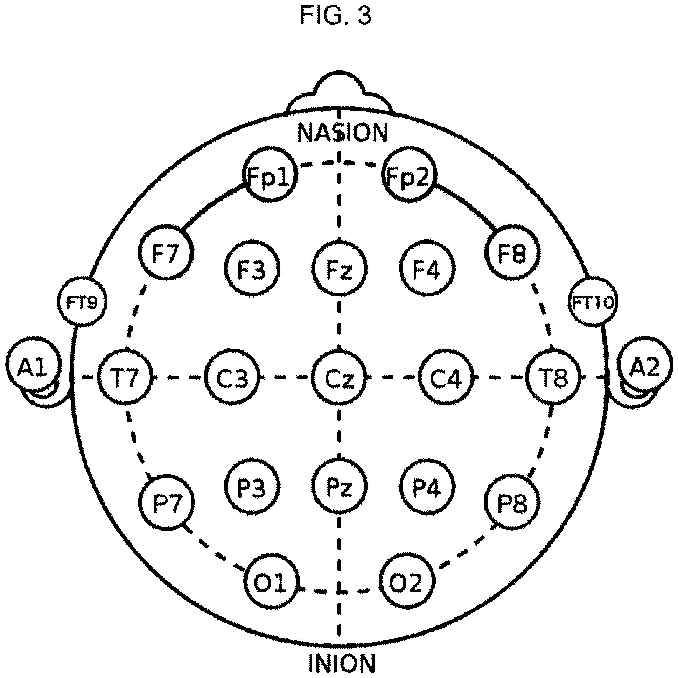

This EEG database is recorded at a sampling frequency of 256 [Hz] and a resolution of 16 bits. The EEG data are saved in EDF (European Data Format) files after power line noise is removed. Fig. 3 shows an electrode arrangement diagram according to the international 10-20 method. In the electrode arrangement diagram shown in Fig. 3, EEG signals from the positions of single-channel electrodes (Fp1-F7, Fp2-F8), which are bipolar derivations from the forehead, are used as channel positions.

Epilepsy has two periods: an "ictal period" during a seizure and an "interictal period" between seizures. The ictal period refers to a time period between the onset of a first symptom and the end of the seizure. During the ictal period, anomaly epileptic EEGs appear in parts or all of the brain. Since the EEG database contains annotation files about seizure start and end times of day identified by clinical researchers, this information was used to identify the ictal period.

The interictal period is defined as a time period after the end of a seizure until the onset of another seizure. This time period accounts for 99[%] of patients' everyday lives. Since some patients experience physical, sensory, or emotional changes before a seizure occurs, a time period immediately preceding a seizure needs to be excluded from the interictal period. Moreover, since patients become delirious after a seizure and regain consciousness gradually, it takes time to return to normal brain activities.

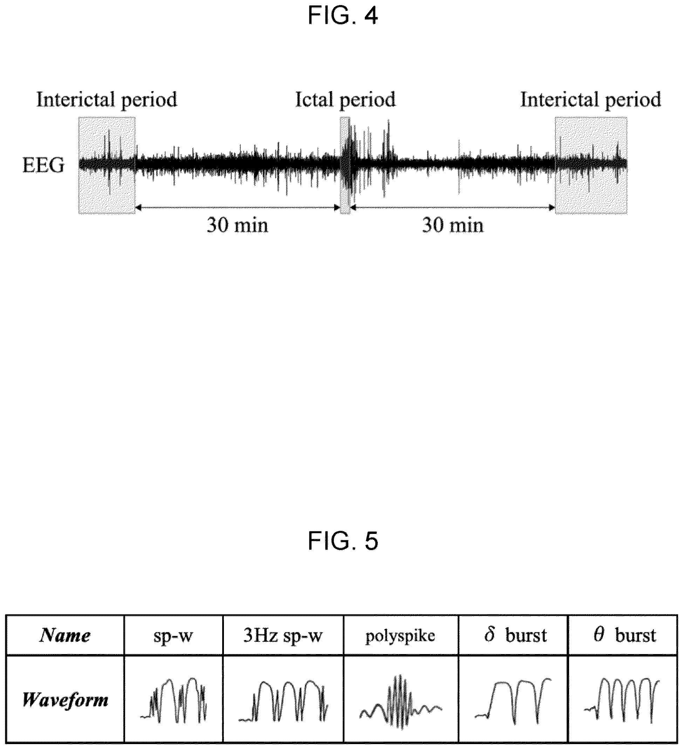

For these reasons, as illustrated in Fig. 4, the interictal period is defined as a time period of 30 minutes or more from the start and end of a seizure. Therefore, in the present invention, the "ictal period" refers to a time period during the seizure and including a specified amount of time (30 minutes) before and after the seizure, and the "interictal period" is defined as a time period after an ictal period until the next ictal period.

The image conversion unit 11 extracts partial time serieses from the EEG signal detected by the signal detection unit 10 while sequentially dividing the EEG signal by a specified time width to make the partial time serieses partially overlap each other in a time direction, and converts each partial time series into a segmented image expressed with a two-dimensional coordinate system whose coordinate axes are time and amplitude.

In the present invention, attention is focused on the shapes of anomaly EEGs which appear during the ictal period. Nerve cells become overly excited by repeated seizure depolarization at an epileptic focus, resulting in epileptic discharges such as spikes and sharp waves. In addition, slow waves occur during hyperpolarization. Since anomaly epileptic EEGs consist of combinations of spikes and slow waves, they can be clearly distinguished from background activities of normal EEGs.

Fig. 5 shows, as a table, the types of anomaly EEGs that appear during epileptic seizures. Spike and slow-wave complexes (sp-w) appear during generalized tonic-clonic seizures, and sp-w of 3 [Hz] appear during absence seizures. EEGs patterns of polyspikes are commonly observed in juvenile myoclonic epilepsy. Moreover, delta bursts and theta bursts are high-amplitude and rhythmic slow waves that occur when the brain functions decline such as loss of consciousness. Accordingly, such characteristic anomaly EEGs patterns occur during each type of seizures, an image recognition algorithm is performed to classify the shapes of normal EEGs and anomaly epileptic EEGs.

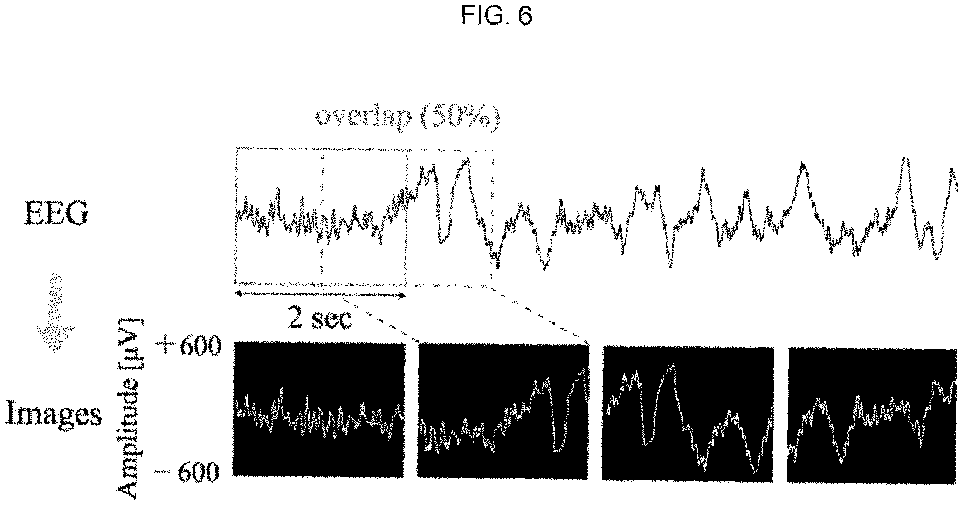

Specifically, as illustrated in Fig. 6, the image conversion unit 11 extracts partial time serieses from the EEG signal detected by the signal detection unit 10 by using a sliding window method while sequentially dividing the EEG signal at two-second intervals with 50[%] overlaps, sets each partial time series in a two-dimensional coordinate system which is enlarged to the range of 6 to 60 times in the amplitude direction, and converts the partial time serieses into segmented images.

Normal scalp EEGs have an amplitude of about 10 to 100 [$\mu$V], and anomaly EEGs often have an amplitude more than twice that of background activities of normal EEGs. So, segmented images of the EEGs were captured within the range of $\pm$ 600 [$\mu$V] to ensure a sufficient area for displaying EEGs. When capturing the images, the background color was set to black and the EEGs were set to white. Moreover, an EEG image was resized to 256 [dpi] $\times$ 256 [dpi], and its central part was cropped to 244 [dpi] $\times$ 244 [dpi]. In addition, since a learned model from a machine learning library (Pytorch torchvision library) was used, an average value and a standard deviation of the RGB level of the ImageNet learned data were used for normalizing the input images.

As a result, with the brain activity detection apparatus 2, when converting the EEG signal to segmented images corresponding to the partial time serieses, data processing was performed to clearly distinguish between normal EEGs and anomaly EEGs and to easily distinguish EEGs patterns from the images, so that the accuracy for extracting EEG characteristic data in each analyzed segmented image can be remarkably improved by the EEG characteristics extraction unit 12 described below.

In particular, noises caused by head movements, facial muscle tensions, blinks, etc. are superimposed on the EEGs and the evaluation in the single sliding window is not sufficient to classify the normal EEGs and the anomaly EEGs, so such insufficiency can be resolved.

The EEG characteristics extraction unit 12 (Fig. 2) sequentially analyzes the segmented images converted by the image conversion unit 11 and extracts the EEG characteristic data in each of the segmented images, respectively.

Specifically, when extracting EEG characteristic data in each segmented image, the EEG characteristics extraction unit 12 uses a specified seizure detection algorithm to sequentially judge the timing to change between the ictal period indicating a time period during a seizure and including a specified amount of time before and after the seizure and the interictal period indicating a time period after an ictal period until the next ictal period.

Since noises caused by head movements, facial muscle tensions, blinks, etc. are superimposed on the EEGs, the evaluation in the single window (the sliding window illustrated in Fig. 6) is not sufficient to classify the normal EEGs and the anomaly EEGs. So, the seizure detection algorithm was used to expand the judgment range.

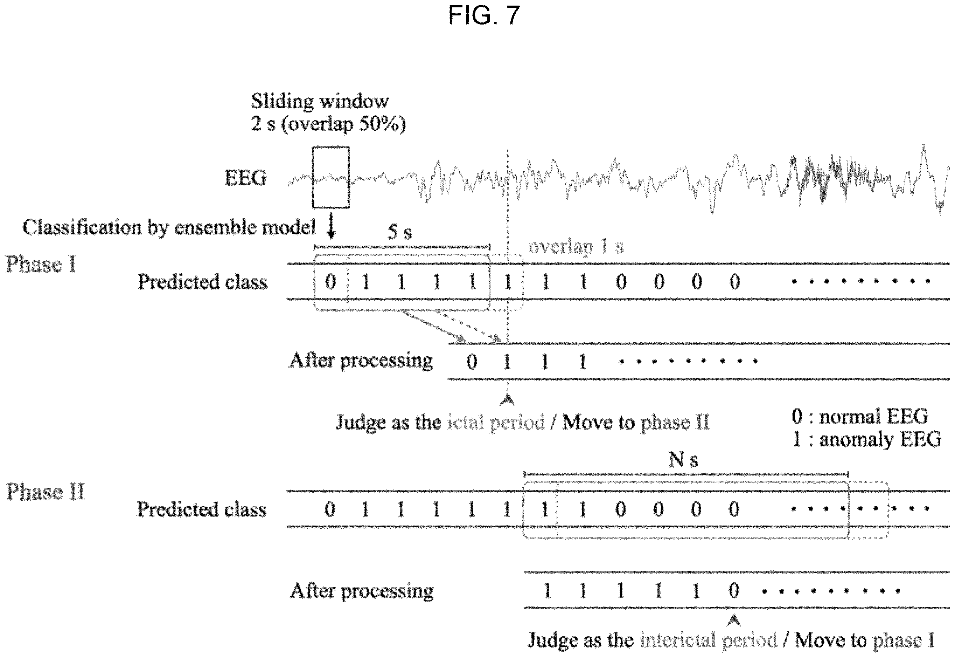

As illustrated in Fig. 7, with the seizure detection algorithm, classes estimated by an ensemble model are defined as "Class 0 (normal)" and "Class 1 (anomaly)" and are composed of two phases (Phase I and Phase II). The role of Phase I is to determine the timing to change from the interictal period to the ictal period, and the role of Phase II is to determine the timing to change from the ictal period to the interictal period.

When Class 1 is counted five times consecutively, our algorithm judges it to be anomaly EEGs and the process proceeds to Phase II. In other cases, it is judged to be normal EEGs. On the other hand, when Class 0 is counted N times consecutively, it is judged to be normal EEGs and the process proceeds to Phase I. In other cases, it is judged to be anomaly EEGs. The N times described above is the number of times that Class 1 is counted after proceeding to Phase II.

As a result, the brain activity detection device 2 can distinguish between the interictal period(s) which accounts for most of test subjects' everyday lives and the ictal period which is the time period during an actual seizure and including a specified amount of time before and after the seizure.

The seizure symptom estimation unit 13 (Fig. 2) estimates the relevant seizure symptom(s) from the EEG characteristic data sequentially extracted by the EEG characteristics extraction unit 12 while referring to a seizure symptom estimation model built by deep learning using, as teacher data, EEG characteristic patterns classified for the respective seizure symptoms caused by human anomaly EEGs.

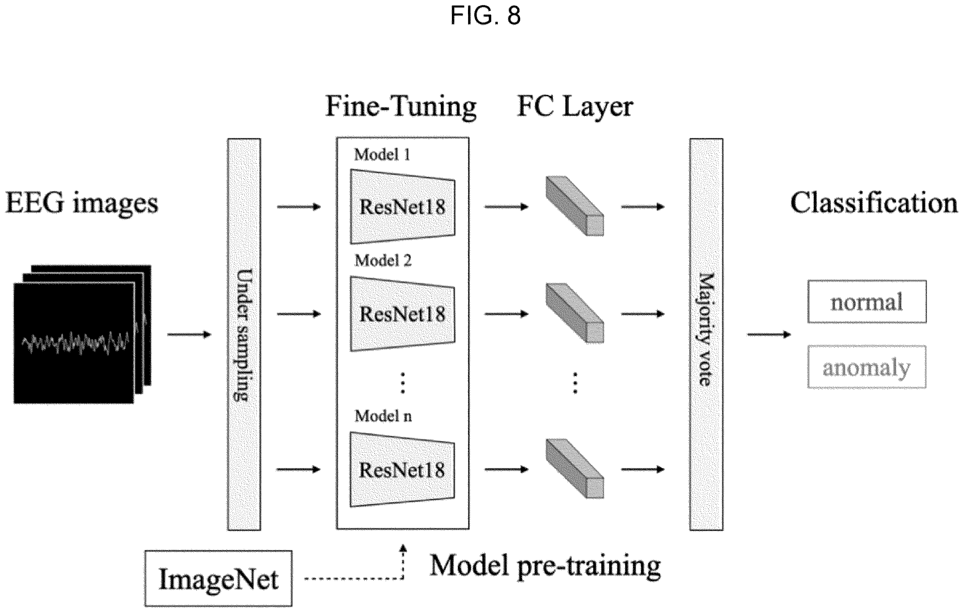

Fig. 8 illustrates the architecture of the seizure symptom estimation model. Since the interictal period(s) accounts for most of patients' everyday lives, the amount of ictal EEG data that can be collected from the patients is very small and a dataset has unbalanced data proportions. Since the classification accuracy becomes low when the dataset with unbalanced data proportions is used for learning with classifiers, it is necessary to balance the data proportions.

Regarding the EEG characteristic data in each of the segmented images extracted by the EEG characteristics extraction unit 12, the seizure symptom estimation unit 13 undersamples the EEG characteristic data corresponding to the interictal period(s) in accordance with the quantity of EEG characteristic data corresponding to the ictal period(s).

Subsequently, the seizure symptom estimation unit 13 creates n subdatasets, and estimates the relevant seizure symptom(s) based on the final output determined by using classifiers corresponding to the respective subdatasets and taking a majority vote of outputs of all the classifiers. Since the amount of data in each subdataset was actually small, a batch size was set to "8."

As a result, with the brain activity detection device 2, the ictal period is very short as compared to the interictal period(s) which accounts for most of the test subjects' everyday lives, so it becomes possible to balance the data proportions obtained during the interictal and ictal periods, thereby making it possible to prevent overfitting (overlearning) in learning by using the classifiers and to prevent degradation of the classification accuracy as a result of the learning.

Moreover, in Fig. 8, in order to detect anomalies of the epileptic EEGs by using the image recognition technology, the seizure symptom estimation unit 13 applies ResNet (Residual Network) 18 as a classifier and applies backpropagation as a method for updating the teacher data of the seizure symptom estimation model.

The residual network is a neural network that can deepen layers and improving the accuracy by introducing shortcut connections. The machine learning library (Pytorch torchvision library) provides five versions (18-layer, 34-layer, 50-layer, 101-layer, and 152-layer) of pre-trained models. The deeper the layers are, the more time it takes to learn the model. So, we decided to use the 18-layer ResNet as the classifier. Incidentally, the weight of the pre-trained ImageNet is read from the torchvision model subpackage.

Furthermore, since the ResNet is a model built to classify input images into 1,000 categories, an output layer needs to be replaced for two-class classification. So, a linear layer having two output nodes was created and was replaced with the output layer of ResNet 18.

With the seizure symptom estimation unit 13, the number of epochs (the number of learning times of each data subset) was set to "50"; and at the time of learning, loss was calculated for each epoch to execute the backpropagation (an error backpropagation method). With the seizure symptom estimation unit 13, at the time of validation, the accuracy for each epoch was calculated by using validation data and a weight parameter of the model was stored when the accuracy improved.

Since the seizure detection is a binary classification task, cross-entropy loss was used as a loss function. Also, Adam (Adaptive Moment Estimation) was used as an optimizer for minimizing the loss function and a learning rate was set to 0.005. In the present invention, the ResNet learned with the ImageNet dataset was fine-tuned for each test subject to create a classifier tailored for the test subject's seizure symptoms.

As a result, with the brain activity detection device 2, by separating an input feature value and a residual feature value in advance by using the residual network, even if the residual feature value cannot be calculated correctly, the feature value supplied from an upstream layer can be transmitted directly without any change to a downstream layer, thereby making it possible to avoid any phenomena that would hinder the progress of learning such as vanishing gradient occurring at each layer on the upstream side of an intermediate layer. As a result, it is possible to suppress degradation of the feature values due to repeated operations associated with deepening of the network.

(3) Experiment Results According to Brain Activity Detection Device

In order to check whether the above-mentioned brain activity detection device 2 can detect anomaly EEGs with sufficient accuracy by using the deep learning method using the seizure symptom estimation model, experiments were conducted on 10 epilepsy patients (3 to 19 years old) registered in the CHB-MIT Scalp EEG database.

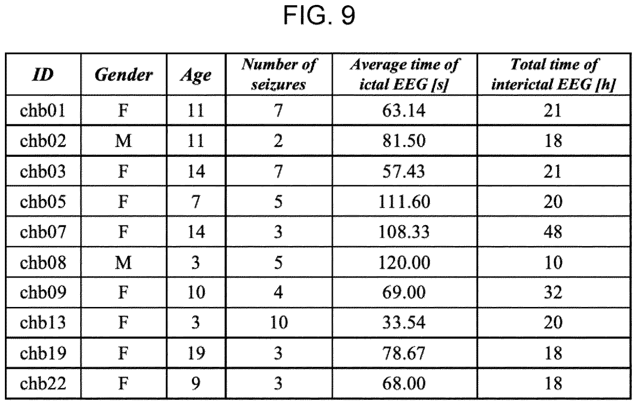

Fig. 9 shows information of epilepsy patients who are test subjects. In Fig. 9, the ID number, gender, age, the number of seizures, average time of the EEG characteristic data corresponding to ictal periods (average time of ictal EEG) [s], and total time of the EEG characteristic data corresponding to interictal periods (total time of interictal EEG) [h] of 10 patients are listed and displayed.

Two types of experiments were conducted, one using the Fp1-F7 channel and the other using the Fp2-F8 channel. In order to evaluate the deep learning method using the seizure symptom estimation model according to the present invention, leave-one-out cross-validation (LOOCV) was performed in which test data of cross-validation was reduced to one.

The experimental procedure is described below. Firstly, N subgroups were created according to the number N of seizures in each patient. Then, after N pieces of seizure data were allocated to subgroups respectively, non-seizure data were allocated to each subgroup so that the total time of the non-seizure data for each subgroup would become equal.

For example, if the ID number is chb01, seven edf files (chb01_03.edf, chb01_04.edf, chb01_15.edf, chb01_16.edf, chb01_18.edf, chb01_21.edf, chb01_26.edf) containing the ictal EEG are included in the dataset as the number of seizures, so that seven subgroups are created and the seizure data are allocated to the subgroups.

Moreover, since the dataset contains 21 hours (the total time of EEG characteristic data corresponding to the interictal periods) of the non-seizure data, the 21 hours of EEG characteristic data was divided into 3 hours of EEG characteristic data, which was allocated to each subgroup. One of the N subgroups was used as validation data, and the others were used as learning data.

This cross-validation was repeated N times, and each of the N subgroups was used as validation data once. Then, the sensitivity, specificity, and detection delay time (DLT) were evaluated.

Now, when TP represents a true positive count, FP represents a false positive count, TN represents a true negative count, and FN represents a true positive count, the sensitivity is expressed by the following expression (1).

[Math. 1]

$$Sensitivity = \frac{TP}{TP + FN} \times 100 \quad \cdots (1)$$

The specificity is expressed by the following expression (2).

[Math. 2]

$$Specificity = \frac{TN}{TN + FP} \times 100 \quad \cdots (2)$$

Moreover, when Ts represents the occurrence time of day of a seizure and Td represents the time of day when the seizure was first detected, the detection delay time (DLT) is expressed by the following equation (3).

[Math. 3]

$$DLT = |T_s - T_d| \quad \cdots (3)$$

In order to determine the type of an epileptic seizure, it is necessary to estimate an epileptic focus by EEGs. In the present invention, 18 channels of EEGs were read from each patient to estimate the epileptic focus. The radiographic image interpretation of EEGs was performed on the basis of phase reversals between the channels, large amplitudes (200 [$\mu$V] or more), appearance of spikes, appearance of sharp waves, and other anomaly epileptic EEGs.

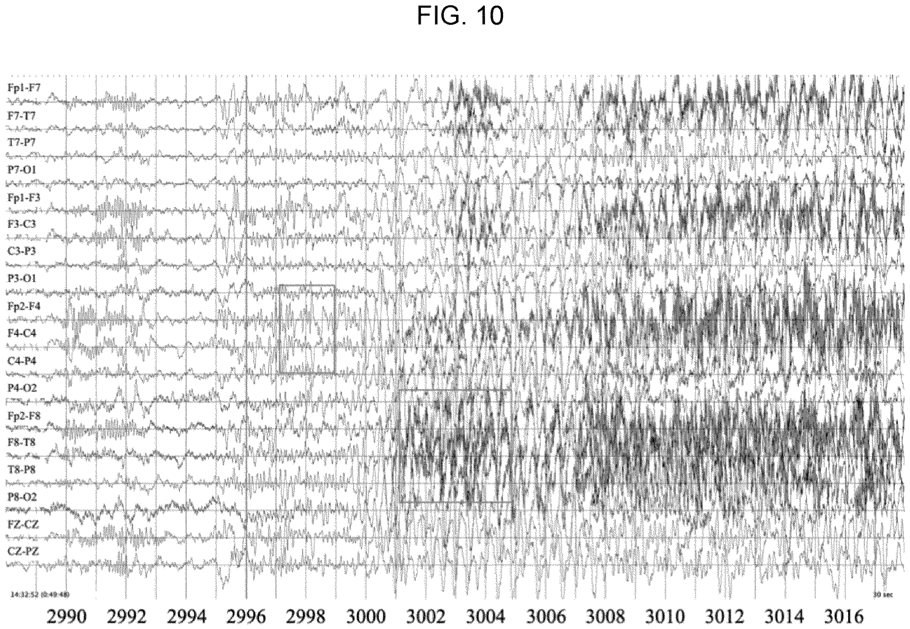

As an example, Fig. 10 shows EEG characteristic data corresponding to the ictal period with the ID number chb01. The seizure started at 2,996 seconds; and immediately after the onset, a high-amplitude phase reversal of EEGs ($\pm$ 200 [$\mu$V]) was observed between Fp2-F4 and F4-C4. Five seconds after the onset of the seizure, polyspikes appeared at Fp2-F8 and F8-T8. These findings suggest that epileptic foci were located around F4 and F8.

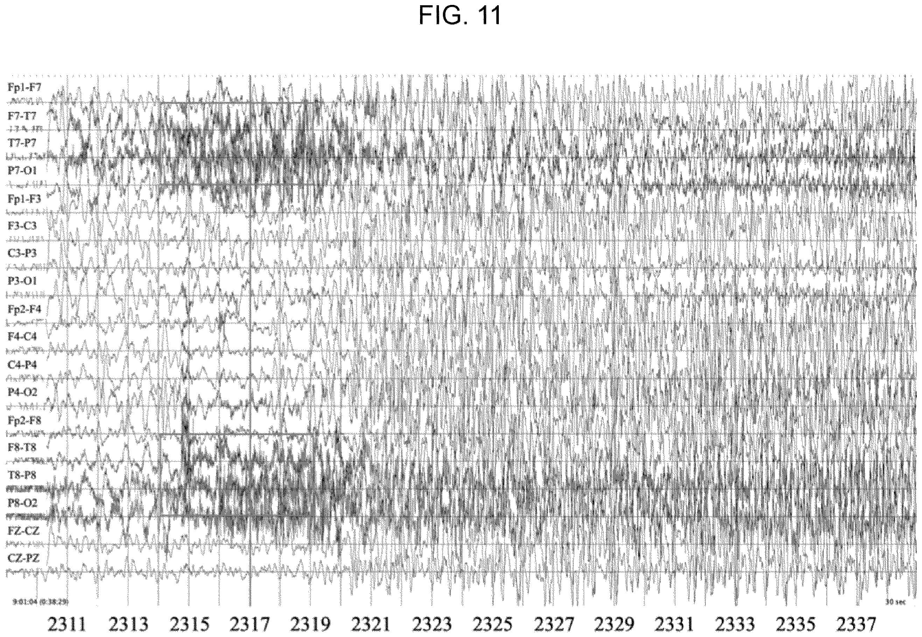

Fig. 11 shows EEG characteristic data corresponding to the ictal period with the ID number chb05. The seizure started at 2,317 seconds; and immediately after the onset, polyspikes appeared between F7-T7 and T7-P7, and between F8-T8 and T8-P8. Moreover, four seconds after the onset of the seizure, complexes of spikes and slow waves were observed all over the head. These findings suggest that seizure foci were located at T7 and T8. So, EEG measurements were performed on all of the 10 patients.

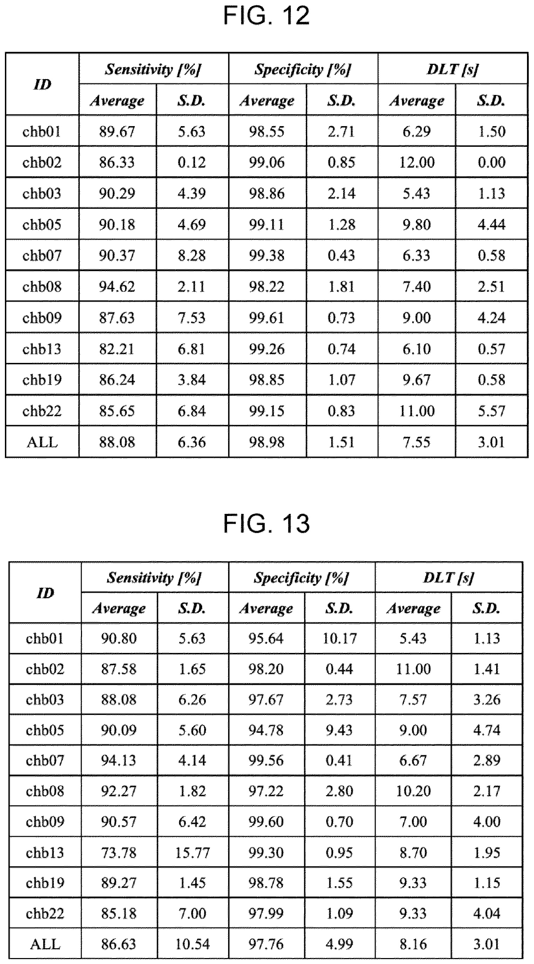

Fig. 12 shows the experiment results using the Fp1-F7 channel (Fig. 3). Average sensitivity was 88.08[%], average specificity was 98.98[%], and average detection delay time (DLT) was 7.55 seconds. Also, Fig. 3 shows the experiment results using the Fp2-F8 channel (Fig. 3). The average sensitivity was 86.63[%], the average specificity was 97.76[%], and the average detection delay time (DLT) was 8.16 seconds.

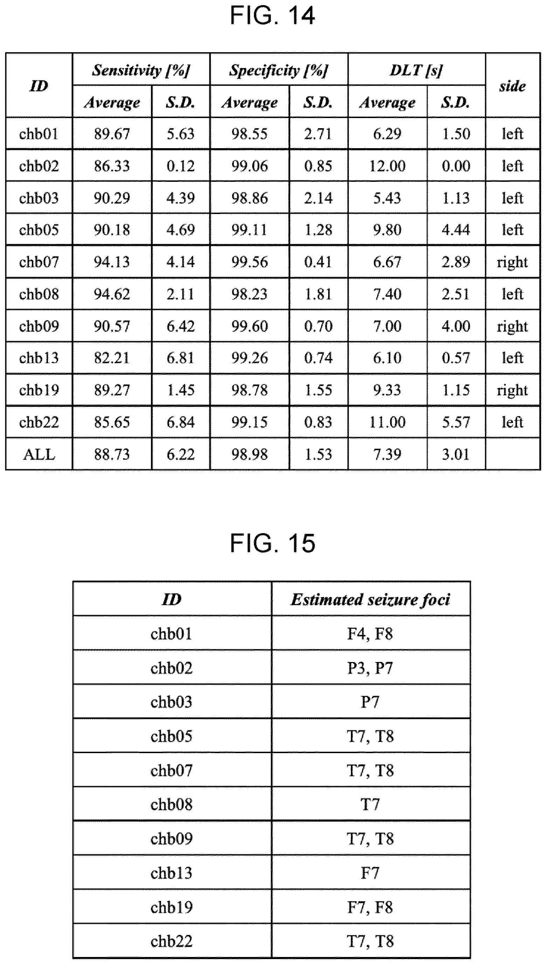

Fig. 14 shows the results of selecting optimum channels in terms of sensitivity and specificity for each patient. The average sensitivity, the average specificity, and the average detection delay time (DLT) were 88.73[%], 98.98[%], and 7.39 seconds, respectively.

Fig. 15 shows the results of radiographic image interpretation of the EEG characteristic data, i.e., the estimated seizure foci, of 10 patients. F7, T7, and P7 indicate anterior, middle, and posterior temporal areas of the left hemisphere respectively, and F8, T8, and P8 indicate anterior, middle, and posterior temporal areas of the right hemisphere (Fig. 3). From the results of the EEG measurements, it was estimated that all the 10 patients had the epileptic foci in the temporal regions of their heads.

(4) Advantageous Effects by This Embodiment

Some methods for detecting seizures by using the multi-channel EEG (electroencephalograms) have been conventionally proposed and such methods can be applied to automatic interpretation of the EEGs and diagnosis of the epilepsy; however, since most of measurement devices are relatively large and electrodes need to be set over the entire head of the test subject, it is very difficult to measure the multi-channel EEG in everyday life.

Therefore, with the brain activity detection device 2 as in the present invention, it was confirmed from the experiment results that the average sensitivity of 88.73[%], which is equivalent to the sensitivity of the conventional method using the multi-channel EEG (average sensitivity of 90.62[%]), can be obtained by detecting seizures using the one-channel frontal lobe EEGs. Since anomaly epileptic EEGs can be clearly distinguished from the background activities of normal EEGs, it is possible to realize extremely sensitive seizure detection based on wave patterns.

Moreover, according to the present invention, the experiment results confirmed that the detection delay time (DLT) was 7.39 seconds. So, since epileptic seizures usually last 30 to 60 seconds, if a caregiver can be notified within 8 seconds after the onset, the caregiver can quickly take care of the test subject after the seizure.

On the other hand, according to the present invention, the average specificity was 98.98[%], which is slightly lower than that of the conventional method using the multi-channel EEG (average sensitivity: 99.32[%]). One reason for this is that there is little brain activity information available from the one-channel EEGs and noises from head movements, facial muscle tensions, blinks, etc. are superimposed on the EEGs. In order to improve the average specificity, it is possible to solve the above by setting a larger window width (overlap ratio) by the sliding window method in the image conversion unit 11 (Fig. 2).

Incidentally, as a result of examining the EEG signal by using the brain activity detection device 2 according to the present invention in order to estimate the positions of the epileptic foci in the patients who are the test subjects, it was estimated that all the 10 patients had epileptic foci in their temporal regions. Therefore, with the brain activity detection device 2 using the one-channel frontal lobe EEGs, it is also possible to detect the temporal lobe seizure(s) in the test subject(s).

There are two types of temporal lobe epilepsies: a "mesial temporal lobe epilepsy" in which seizures are caused by rigidity of the limbic hippocampus; and a "lateral temporal lobe epilepsy" in which seizures are caused by epileptic discharges in the cerebral neocortex. Since the frontal association area is interconnected via fibers with the temporal association area and the limbic system, when an epileptic discharge occurs in the temporal region, it is assumed that abnormal excitations propagate to the frontal association area.

Studies have shown that using the one-channel temporal lobe EEGs (FT10-T8) is effective in detecting temporal lobe seizures; however, when measuring temporal channels, it is often difficult to place the electrodes at measurement points due to the presence of the test subject's hair.

Therefore, the method of detecting the one-channel frontal lobe EEGs from the test subject's forehead using the brain activity detection device 2 of the present invention is more effective in detecting the temporal lobe seizures during everyday life in terms of easiness of placing the electrodes on the test subject's head, as compared to other methods using the one-channel EEGs.

According to the configuration described above, it is possible to implement the brain activity detection device 2 that can effectively detect seizures caused by the test subject's brain activities in everyday life by using a method of measuring the one-channel frontal EEGs.

(5) Other Embodiments

Incidentally, this embodiment has described, as explained above, the case where the seizure symptom estimation unit 13 in the brain activity detecting device 2 estimates the relevant seizure symptom(s) by the supervised deep learning using the seizure symptom estimation model; however, the present invention is not limited to this example and a neural network(s) other than the residual network(s) may be applied as a classifier(s) in the deep learning. Moreover, an update method other than the backpropagation method may be applied as an update method of the teacher data of the seizure symptom estimation model.

Furthermore, this embodiment has described the case where the image conversion unit 11 extracts partial time serieses from the EEG signal detected by the signal detection unit 10 by using the sliding window method while sequentially dividing the EEG signal at two-second intervals with 50[%] overlaps, and sets each partial time series in the two-dimensional coordinate system which is enlarged to the range of 6 to 60 times in the amplitude direction, and converts the partial time serieses into the segmented images; however, the present invention is not limited to this example and, if the EEG characteristic data in the segmented images can be effectively extracted by the EEG characteristics extraction unit 12, the overlap ratio may be 50[%] or more, segmentation time may be set to other than 2 seconds, and the amplitude increase ratio does not have to be limited to 6 to 60 times.

REFERENCE SIGNS LIST

1:      brain activity detection system
2:      brain activity detection device
2A:     electrodes
3:      terminal device
4:      network
5:      cloud server
6:      data analysis infrastructure
10:     signal detection unit
11:     image conversion unit
12:     EEG characteristics extraction unit
13:     seizure symptom estimation unit
14:     transmission unit

**Claims**

1.  A brain activity detection device comprising:

    a signal detection unit that detects an EEG signal of a single channel indicating frontal lobe EEGs via a pair of electrodes placed at a test subject's forehead;
    an image conversion unit that extracts partial time serieses from the EEG signal detected by the signal detection unit while sequentially dividing the EEG signal by a specified time width to make the partial time serieses partially overlap each other in a time direction, and performs data conversion of the respective partial time serieses to segmented images expressed with a two-dimensional coordinate system whose coordinate axes are time and amplitude;
    an EEG characteristics extraction unit that sequentially analyzes the segmented images converted by the image conversion unit and extracts each piece of EEG characteristic data in each of the segmented images; and
    a seizure symptom estimation unit that, while referring to a seizure symptom estimation model constructed by deep learning by using, as teacher data, EEG characteristic patterns classified according to respective seizure symptoms caused by human anomaly EEGs, estimates a relevant seizure symptom from the EEG characteristic data sequentially extracted by the EEG characteristics extraction unit.

2.  The brain activity detection device according to claim 1,
    wherein the image conversion unit extracts the partial time serieses from the EEG signal detected by the signal detection unit by using a sliding window method, while sequentially dividing the EEG signal by a specified time interval with overlaps at a specified ratio, and sets the respective partial time serieses in the two-dimensional coordinate system, which is enlarged within a range of a specified multiple of the extracted partial time serieses in an amplitude direction, and converts the partial time serieses to the segmented images.

3.  The brain activity detection device according to claim 1 or 2,
    wherein the EEG characteristics extraction unit sequentially judges a timing to change between an ictal period indicating a time period during a seizure and including a specified amount of time before and after the seizure and an interictal period indicating a time period after the ictal period until a next ictal period by using a specified seizure detection algorithm when extracting each piece of the EEG characteristic data in each of the segmented images.

4.  The brain activity detection device according to claim 3,
    wherein the seizure symptom estimation unit undersamples the EEG characteristic data corresponding to the interictal period in accordance with a quantity of the EEG characteristic data corresponding to the ictal period with respect to the EEG characteristic data in the respective segmented images extracted by the EEG characteristics extraction unit, then creates n pieces of subdatasets, and estimates the relevant seizure symptom, by using each of classifiers according to each piece of the subdatasets, based on final output decided by taking a majority vote of outputs from all the classifiers.

5.  The brain activity detection device according to claim 4,

wherein the seizure symptom estimation unit applies a residual network as the classifier and applies backpropagation as a method for updating the teacher data for the seizure symptom estimation model.

6. A brain activity detection method comprising:

a first step of detecting an EEG signal of a single channel indicating frontal lobe EEGs via a pair of electrodes placed at a test subject's forehead;

a second step of extracting partial time serieses from the EEG signal detected by the first step while sequentially dividing the EEG signal by a specified time width to make the partial time serieses partially overlap each other in a time direction, and performing data conversion of the respective partial time serieses to segmented images expressed with a two-dimensional coordinate system whose coordinate axes are time and amplitude;

a third step of sequentially analyzing the segmented images converted by the second step and extracting each piece of EEG characteristic data in each of the segmented images; and

a fourth step of, while referring to a seizure symptom estimation model constructed by deep learning by using, as teacher data, EEG characteristic patterns classified according to respective seizure symptoms caused by human anomaly EEGs, estimating a relevant seizure symptom from the EEG characteristic data sequentially extracted by the third step.

7. The brain activity detection method according to claim 6,
wherein in the second step, the partial time serieses are extracted from the EEG signal detected by the first step by using a sliding window method, while sequentially dividing the EEG signal by a sliding window at specified time intervals with overlaps at a specified ratio, and the respective partial time serieses are set in the two-dimensional coordinate system, which is enlarged within a range of a specified multiple of the extracted partial time serieses in an amplitude direction, and are converted to the segmented images.

8. The brain activity detection method according to claim 6 or 7,
wherein in the third step, a timing to change between an ictal period indicating a time period during a seizure and including a specified amount of time before and after the seizure and an interictal period indicating a time period after the ictal period until a next ictal period is sequentially judged by using a specified seizure detection algorithm when extracting each piece of the EEG characteristic data in each of the segmented images.

9. The brain activity detection method according to claim 8,
wherein in the fourth step, the EEG characteristic data corresponding to the interictal period are undersampled in accordance with a quantity of the EEG characteristic data corresponding to the ictal period with respect to the EEG characteristic data in the respective segmented images extracted by the third step, n pieces of subdatasets are then created, and the relevant seizure symptom are estimated by using each of classifiers according to each piece of the subdatasets, based on final output decided by taking a majority vote of outputs from all the classifiers.

10. The brain activity detection method according to claim 9,
wherein in the fourth step, a residual network is applied as the classifier and backpropagation is applied as a method for updating the teacher data for the seizure symptom estimation model.

# FIG. 1

1 Brain Activity Detection System

# FIG. 2

FIG. 3

# FIG. 4

Interictal period       Ictal period       Interictal period

EEG

30 min       30 min

# FIG. 5

| *Name* | sp-w | 3Hz sp-w | polyspike | $\delta$ burst | $\theta$ burst |
|--------|------|----------|-----------|----------------|----------------|
| *Waveform* | | | | | |

FIG. 6

overlap (50%)

EEG

Images

2 sec

Amplitude [µV]

+600

−600

EP 4 437 964 A1

# FIG. 7

Sliding window
2 s (overlap 50%)

EEG

Classification by ensemble model

Phase I

Predicted class

5 s

overlap 1 s

0  1  1  1  1  1  1  1  0  0  0  0  ·············

After processing

0  1  1  1  ·············

Judge as the ictal period / Move to phase II

0 : normal EEG
1 : anomaly EEG

Phase II

Predicted class

N s

0  1  1  1  1  1  1  1  0  0  0  0  ····  ··  ···

After processing

1  1  1  1  1  0  ············

Judge as the interictal period / Move to phase I

EP 4 437 964 A1

# FIG. 8

Fine-Tuning  FC Layer

EEG images → Under sampling → [Model 1: ResNet18, Model 2: ResNet18, ..., Model n: ResNet18] → Majority vote → Classification: normal / anomaly

ImageNet ----→ Model pre-training

EP 4 437 964 A1

# FIG. 9

| ID | Gender | Age | Number of seizures | Average time of ictal EEG [s] | Total time of interictal EEG [h] |
|---|---|---|---|---|---|
| chb01 | F | 11 | 7 | 63.14 | 21 |
| chb02 | M | 11 | 2 | 81.50 | 18 |
| chb03 | F | 14 | 7 | 57.43 | 21 |
| chb05 | F | 7 | 5 | 111.60 | 20 |
| chb07 | F | 14 | 3 | 108.33 | 48 |
| chb08 | M | 3 | 5 | 120.00 | 10 |
| chb09 | F | 10 | 4 | 69.00 | 32 |
| chb13 | F | 3 | 10 | 33.54 | 20 |
| chb19 | F | 19 | 3 | 78.67 | 18 |
| chb22 | F | 9 | 3 | 68.00 | 18 |

FIG. 10

EP 4 437 964 A1

FIG. 11

# FIG. 12

| ID | Sensitivity [%] | | Specificity [%] | | DLT [s] | |
|---|---|---|---|---|---|---|
| | Average | S.D. | Average | S.D. | Average | S.D. |
| chb01 | 89.67 | 5.63 | 98.55 | 2.71 | 6.29 | 1.50 |
| chb02 | 86.33 | 0.12 | 99.06 | 0.85 | 12.00 | 0.00 |
| chb03 | 90.29 | 4.39 | 98.86 | 2.14 | 5.43 | 1.13 |
| chb05 | 90.18 | 4.69 | 99.11 | 1.28 | 9.80 | 4.44 |
| chb07 | 90.37 | 8.28 | 99.38 | 0.43 | 6.33 | 0.58 |
| chb08 | 94.62 | 2.11 | 98.22 | 1.81 | 7.40 | 2.51 |
| chb09 | 87.63 | 7.53 | 99.61 | 0.73 | 9.00 | 4.24 |
| chb13 | 82.21 | 6.81 | 99.26 | 0.74 | 6.10 | 0.57 |
| chb19 | 86.24 | 3.84 | 98.85 | 1.07 | 9.67 | 0.58 |
| chb22 | 85.65 | 6.84 | 99.15 | 0.83 | 11.00 | 5.57 |
| ALL | 88.08 | 6.36 | 98.98 | 1.51 | 7.55 | 3.01 |

# FIG. 13

| ID | Sensitivity [%] | | Specificity [%] | | DLT [s] | |
|---|---|---|---|---|---|---|
| | Average | S.D. | Average | S.D. | Average | S.D. |
| chb01 | 90.80 | 5.63 | 95.64 | 10.17 | 5.43 | 1.13 |
| chb02 | 87.58 | 1.65 | 98.20 | 0.44 | 11.00 | 1.41 |
| chb03 | 88.08 | 6.26 | 97.67 | 2.73 | 7.57 | 3.26 |
| chb05 | 90.09 | 5.60 | 94.78 | 9.43 | 9.00 | 4.74 |
| chb07 | 94.13 | 4.14 | 99.56 | 0.41 | 6.67 | 2.89 |
| chb08 | 92.27 | 1.82 | 97.22 | 2.80 | 10.20 | 2.17 |
| chb09 | 90.57 | 6.42 | 99.60 | 0.70 | 7.00 | 4.00 |
| chb13 | 73.78 | 15.77 | 99.30 | 0.95 | 8.70 | 1.95 |
| chb19 | 89.27 | 1.45 | 98.78 | 1.55 | 9.33 | 1.15 |
| chb22 | 85.18 | 7.00 | 97.99 | 1.09 | 9.33 | 4.04 |
| ALL | 86.63 | 10.54 | 97.76 | 4.99 | 8.16 | 3.01 |

## FIG. 14

| ID | Sensitivity [%] | | Specificity [%] | | DLT [s] | | side |
|---|---|---|---|---|---|---|---|
| | Average | S.D. | Average | S.D. | Average | S.D. | |
| chb01 | 89.67 | 5.63 | 98.55 | 2.71 | 6.29 | 1.50 | left |
| chb02 | 86.33 | 0.12 | 99.06 | 0.85 | 12.00 | 0.00 | left |
| chb03 | 90.29 | 4.39 | 98.86 | 2.14 | 5.43 | 1.13 | left |
| chb05 | 90.18 | 4.69 | 99.11 | 1.28 | 9.80 | 4.44 | left |
| chb07 | 94.13 | 4.14 | 99.56 | 0.41 | 6.67 | 2.89 | right |
| chb08 | 94.62 | 2.11 | 98.23 | 1.81 | 7.40 | 2.51 | left |
| chb09 | 90.57 | 6.42 | 99.60 | 0.70 | 7.00 | 4.00 | right |
| chb13 | 82.21 | 6.81 | 99.26 | 0.74 | 6.10 | 0.57 | left |
| chb19 | 89.27 | 1.45 | 98.78 | 1.55 | 9.33 | 1.15 | right |
| chb22 | 85.65 | 6.84 | 99.15 | 0.83 | 11.00 | 5.57 | left |
| ALL | 88.73 | 6.22 | 98.98 | 1.53 | 7.39 | 3.01 | |

## FIG. 15

| ID | Estimated seizure foci |
|---|---|
| chb01 | F4, F8 |
| chb02 | P3, P7 |
| chb03 | P7 |
| chb05 | T7, T8 |
| chb07 | T7, T8 |
| chb08 | T7 |
| chb09 | T7, T8 |
| chb13 | F7 |
| chb19 | F7, F8 |
| chb22 | T7, T8 |

## INTERNATIONAL SEARCH REPORT

| International application No. |
|---|
| **PCT/JP2022/036874** |

### A. CLASSIFICATION OF SUBJECT MATTER

*A61B 5/372*(2021.01)i
FI: A61B5/372

According to International Patent Classification (IPC) or to both national classification and IPC

### B. FIELDS SEARCHED

Minimum documentation searched (classification system followed by classification symbols)

A61B5/372

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | US 2011/0270117 A1 (GLKK, INC.) 03 November 2011 (2011-11-03)<br>paragraphs [0033]-[0065], fig. 1-6 | 1-10 |
| Y | JP 1-293842 A (NISHIMURA, Toshihiro) 27 November 1989 (1989-11-27)<br>p. 4, upper left column, line 14 to p. 7, lower right column, line 11, fig. 1-9 | 1-10 |
| Y | WO 2019/092836 A1 (FUJITSU LTD) 16 May 2019 (2019-05-16)<br>paragraphs [0028]-[0061], fig. 1-7 | 1-10 |
| Y | BANCAUD, Jean et al. Proposal for Revised Clinical and Electroencephalographic<br>Classification of Epileptic Seizures. Epilepsia. 1981, 22, pp. 489-501<br>I. PARTIAL (FOCAL, LOCAL) SEIZURES to IV. ADDENDUM | 1-10 |

☐ Further documents are listed in the continuation of Box C.        ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **01 December 2022** | **13 December 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

### INTERNATIONAL SEARCH REPORT
Information on patent family members

International application No.

**PCT/JP2022/036874**

| Patent document cited in search report | | | Publication date (day/month/year) | Patent family member(s) | | | Publication date (day/month/year) |
|---|---|---|---|---|---|---|---|
| US | 2011/0270117 | A1 | 03 November 2011 | US 2013/0338449 A1 paragraphs [0033]-[0065], fig. 1-6 | | | |
| JP | 1-293842 | A | 27 November 1989 | (Family: none) | | | |
| WO | 2019/092836 | A1 | 16 May 2019 | US 2020/0279408 A1 paragraphs [0044]-[0083], fig. 1-7 | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017080483 A

- JP 2020512860 W

**Non-patent literature cited in the description**

- **ZABIHI M ; KIRANYAZ S ; INCE T ; GABBOUJ M.** *Patient-specific epileptic seizure detection in long-term eeg recording in paediatric patients with intractable seizures,* 2013